# EUROPÄISCHE PATENTANMELDUNG

(11) **EP 0 764 845 A2**
(43) Veröffentlichungstag der Anmeldung: **26.03.1997**
(21) Anmeldenummer: 96114072.0
(22) Anmeldetag: 03.09.1996
(51) Int. Cl.: G01N 21/88

(54) **Einrichtung zum Erfassen von Fehlstellen auf einer glatten Oberfläche**

(30) Priorität: 19.09.1995 DE 19534716
(71) Anmelder: AUTRONIC Gesellschaft für Bildverarbeitung und Systeme mbH, D-76229 Karlsruhe (DE)
(72) Erfinder: Drixler, Bernhard, Dipl.-Ing., 76676 Graben-Neudorf (DE)
(74) Vertreter: Hofmann, Gerhard, Dipl.-Ing. Patentassessor

(57) **Zusammenfassung**

Um bei der optischen Inspektion einer an sich ebenen Oberfläche (12) mechanische Fehlstellen (Einsenkungen und Erhebungen), die bei schräger Bestrahlung (16) zu Schlagschatten (19) führen, von entsprechend kontrastreichen Oberflächen-Musterungen (wie der Maserung von Holz) unterscheiden zu können, erfolgt eine Bestrahlung (16) gleichzeitig aus unterschiedlichen Richtungen, beispielsweise mit unterschiedlichen Lichtfarben. Die von der ebenen Oberfläche (12) - unabhängig von einem darin etwa enthaltenen kontrastreichen Muster - ausgehenden Mischlicht-Reflexe (M) können für die Weiterverarbeitung in einer Farb-Kamera (17) durch Vergleich der beiden Farbkomponenten neutralisiert werden. Der für die eine Bestrahlungsrichtung auftretende Schlagschatten (19) wird dagegen aus der ersterer gegengesetzten Richtung unifarbig ausgeleuchtet. Dadurch liefern die an Fehlstellen-Rändern (20) auftretenden Schattenbereiche (19) im wesentlichen nur Reflexionen in einer Farbe, deren Sensor-Signale sich beim Vergleich, etwa durch Farbkomponenten-Subtraktion oder -Verhältnisbildung, nicht kompensieren und somit für die weitere Güteklassifizierung eine eindeutige Information über das Vorliegen einer Fehlstelle in der ansonsten glatten, auch kontrastreich gemusterten, Oberfläche (12) liefern.

## Beschreibung

Erfindung betrifft eine Einrichtung gemäß dem Oberbegriff des Anspruches 1.

Eine solche Einrichtung ist aus der EP 0 634 648 A1 für die optronische Oberflächeninspektion bekannt, bei der es insbesondere um die vollautomatische Güteklassifizierung von gehobelten Brettern nach Maßgabe der Anzahl und Ausdehnung von Ästen, Harzgallen, Verfärbungen, Hobelbrand oder dergleichen Fehlstellen geht. Dafür wird ein Bildaufnehmer in Form einer Grauwert-Zeilenkamera auf die quer zu ihrem Erfassungsbereich vorbewegte Oberfläche gerichtet, welche ihrerseits mit Halogenscheinwerfern angestrahlt wird, um die durch signifikante Kontrastsprünge gegenüber der Umgebung gekennzeichneten Fehlstellen nach Ausdehnung und Anzahl erfassen zu können. Damit dabei auch ein glattes Loch (insbesondere ein Astloch) erfaßt wird, erfolgt die Bestrahlung unter spitzem Winkel gegenüber der Holz-Oberfläche, so daß am bestrahlungsseitigen Rand des Loches ein deutlicher, sichelförmiger Schattenwurf auftritt.

Für die automatische Güte-Sortierung von gehobeltem Profilholz hat diese vorbekannte Einrichtung sich in der Praxis bereits hervorragend bewährt. Nicht ohne weiteres einsetzbar ist sie allerdings, wenn es nicht um die Erfassung von Fehlstellen in Form von kontrastierenden Mustern auf der Werkstück-Oberfläche geht, sondern um Fehler in Form von Unebenheiten in einer an sich glatten, ebenen und insbesondere gemusterten Oberfläche. Die Unebenheiten können durch Werkzeugfehler hervorgerufene rauhe Bereiche in einer ansonsten glatt-gehobelten und durch die Maserung strukturierten Holzoberfläche sein, oder aber Herstellungs- bzw. Bearbeitungsfehler an metallenen oder Kuststoff-Gußstücken. Solche Oberflächen-Unebenheiten führen zwar bei schräger Beleuchtung ebenfalls zu einem deutlich gegenüber der Umgebung kontrastierenden Schattenwurf; diese Schattenbilder sind aber kaum zu unterscheiden von anderen, die Oberflächenqualität nicht beeinträchtigenden Mustern wie der Maserung des Holzes oder einer Bedruckung bzw. Beschichtung von Oberflächen. Solche Muster würden bei einer Inspektion mittels der gattungsgemäßen Einrichtung zu Fehlerinformationen führen, obgleich sie auf einer glatten und somit nicht zu beanstandenden Oberfläche vorkommen, also bei der Qualitätsüberwachung nicht zu einer Herabstufung oder Fehlermeldung führen dürfen.

In Erkenntnis dieser Gegebenheiten liegt vorliegender Erfindung die technische Problematik zugrunde, einer Einrichtung gattungsgemäßer Art dahingehend weiterzubilden, daß die Fehlstellen-Inspektion nur Oberflächenfehler aber nicht Oberflächenmuster erfaßt.

Diese Aufgabe ist erfindungsgemäß im wesentlichen dadurch gelöst, daß die gattungsgemäße Einrichtung auch gemäß dem Kennzeichnungsteil des Hauptanspruches ausgelegt ist.

Nach dieser Lösung erfaßt der Bildaufnehmer aus dem Inspektionsbereich auf einer glatten, gewöhnlich ebenen Oberfläche reflektiertes Licht von zwei Bestrahlungsrichtungen, also Mischlicht aus beiden prinzipiell gleich intensiven Bestrahlungsquellen. Wenn ein Oberflächenfehler (d. h. eine Erhöhung oder eine Vertiefung in der glatten Umgebung) für die eine Bestrahlungsrichtung zum Schattenwurf führt, dann wird dieser abgeschattete Bereich aber von der entgegengesetzten Bestrahlungsrichtung noch erfaßt. Der Bildaufnehmer empfängt also aus dem Abschattungsbereich eines Streiflicht-Randes im wesentlichen nur die Uni-Reflexion einer der beiden Bestrahlungsquellen. Das läßt sich prinzipiell dadurch signaltechnisch auswerten, daß bei Mischlicht-Empfang die beiden Bestrahlungskomponenten weitgehend einander kompensieren, während in der Reflexion aus einem Abschattungsbereich signifikanterweise nur eine der beiden Bestrahlungen ganz maßgeblich enthalten ist. Das ist die Fehlerinformation, die dann der Musterverarbeitung zur Fehlerklassifikation unterzogen werden kann. Selbst kontrastreiche Muster in der Oberfläche führen nicht zu solchen Fehlerinformationen, weil unabhängig von der Mustergeometrie die glatte Oberfläche ein Mischlicht aus beiden Bestrahlungsrichtungen reflektiert.

Das aber wird nicht erreicht, wenn gemäß der DE-OS 41 07 701 ein hinsichtlich seiner Konturen zu erfassendes Brett aus unterschiedlichen Richtungen in unterschiedlichen Farben bestrahlt wird, die nacheinander über Filter auf den Streulicht-Detektor geführt werden, um eine Auswertung von Mischstrahlung zu vermeiden.

Das erfindungsgemäße Ergebnis wird auch dann nicht erreicht, wenn gemäß der DE 28 43 257 A1 ein Brett, dessen Breite zwischen Waldkanten gemessen werden soll, geneigt zur Oberfläche des Brettes von einander unterscheidbare Bestrahlungen erfährt; wobei dort zwischen dem Brett und dem Zeilenarray-Bildaufnehmer zwei Filter angeordnet sind, um den Bildaufnehmer so gut wie möglich gegen Uni-Reflexionen von den beiden Waldkanten abzuschirmen und somit nur die dann beiderseits scharfberandete Mischlicht-Reflexion (an der Brett-Oberfläche zwischen den beiden Waldkanten) auf den Bildaufnehmer einwirken zu lassen. Das bedingt, daß die zwischen den beiden Waldkanten nutzbare Breite des Brettes einigermaßen bekannt und konstant sein muß, um die Filter (zur Blockade der jeweils dem Filter zugewandten Uni-Strahlung) strahlengeometrisch korrekt gegenüber der Kante versetzt positionieren zu können. Aufgrund nicht idealer Filtercharakteristiken strahlen allerdings auch Uni-Reflexionen von den Waldkanten auf den Bildaufnehmer durch und führen so zu weniger kontrastreichen Kanten beiderseits der Mischlicht-Ränder, deren gegenseitiger Abstand vom Bildaufnehmer ausgemessen werden soll. Etwaige, für die dortige Messung nicht interessierende, Unebenheiten in der Plattenoberfläche, die zu lokalen Uni-Schlagschatten im Mischlichtbereich führen, werden je nach ihrem Abstand vom Rand des Brettes mehr oder weniger vom Filter abgeblockt, jedenfalls im Bildaufnehmer nicht ausgewertet. Die unterschiedlichen, flach gegenüber der Oberfläche des Brettes geneigten und gegeneinander gerichteten Bestrahlungen können sich voneinander durch ihre Frequenz oder durch ihre Modulation unterscheiden. Stets geht es darum, je eine möglichst scharfe Mischreflexions-Kante längs beiden Seiten des Brettes dadurch hervorzurufen, daß die außen benachbarten Unireflexionen möglichst gut ausgefiltert werden, also den Bildaufnehmer möglichst nicht erreichen. Schon deshalb kann jener Bildaufnehmer keine Information über Oberflächenfehler liefern; außerdem müßte für eine Oberflächeninformation der Schwerpunkts-Abstand der Filter ständig variiert werden, um hinsichtlich ihrer Abmessungen und Lage auf dem Brett vorher nicht bekannte Oberflächenfehler nach der Methode vorliegender Erfindung erfassen zu können, worauf sich aber aus jener Publikation keinerlei Hinweis ergibt.

Nichts mit der Problematik der Unterscheidung zwischen stark kontrastierenden Oberflächenmustern und mechanischen Oberflächenfehlern zu tun hat es, gemäß der DE 35 35 400 A1 ein Prüfobjekt so in zwei Teile zu zerlegen, daß sich komplementär-symmetrische Hälften ergeben - um aus einer Störung dieser Symmetrie auf einen Fehler zu schließen. Der Fehler kann ein Berandungsfehler oder ein Oberflächenfehler sein. Jedoch ist nicht erkennbar, ob es sich beim Oberflächenfehler um einen Musterungsfehler oder um einen mechanischen Fehler handelt, welch letzterer allein Gegenstand der Qualitätsbeurteilung gemäß vorliegender Erfindung sein soll.

Im für die Praxis einfachsten Falle erfolgen auch bei vorliegender Erfindung die beiden entgegengesetzt orientierten geneigten Oberflächenbestrahlungen mit Scheinwerfern unterschiedlicher Lichtfarbe, so daß in einer Farbkamera ein Vergleich der Farbanteile im aufgenommenen Mischlicht nur dann zu einem Ausgangs- oder Fehlersignal führt, wenn im reflektierten Mischlicht nicht beide Bestrahlungsfarben wieder wenigstens angenähert gleich kräftig enthalten sind. Statt mit Strahlung unterschiedlicher Wellenlänge (Farbe) kann aber die Bestrahlung auch beispielsweise in unterschiedlicher Polarisierung oder mit unterschiedlichen Laser-Modes erfolgen, so daß dann in dem Bildaufnehmer über einen Analysator festgestellt wird, ob beide Polarisationsrichtungen bzw. Modes gleichzeitig aufgenommen werden (weil sie an einer glatten Oberfläche reflektiert wurden), oder ob eine signifikant überwiegt (weil die andere durch eine Unebenheit abgeschattet ist).

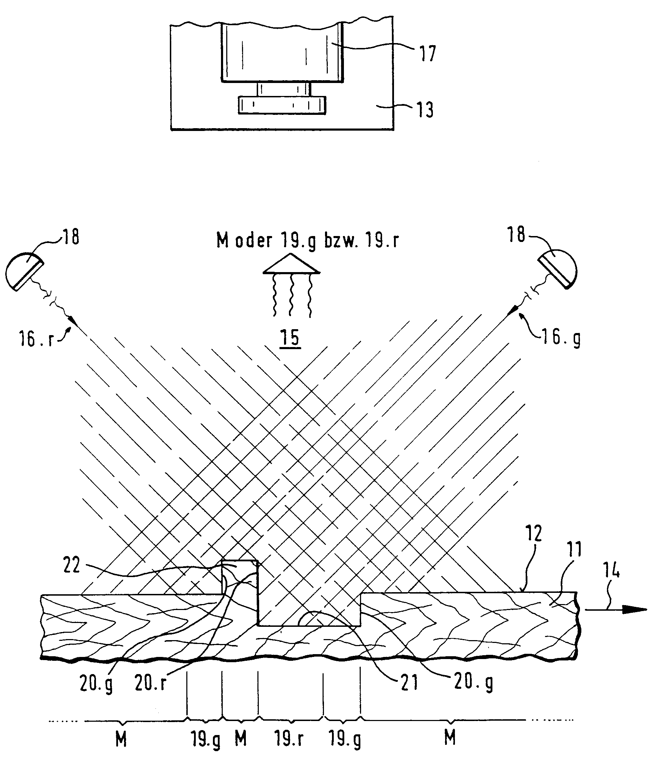

Zusätzliche Alternativen und Weiterbildungen sowie weitere Merkmale und Vorteile der Erfindung ergeben sich aus den weiteren Ansprüchen und, auch unter Berücksichtigung der Zusammenfassung am Ende, aus nachstehender Beschreibung eines in der Zeichnung unter Beschrenkung auf das Wesentliche stark abstrahiert und nicht maßstabsgerecht skizzierten bevorzugten Realisierungsbeispiels zur erfindungsgemäßen Lösung. Die einzige Figur der Zeichnung zeigt im Längsschnitt einen Bildaufnehmer zum Erfassen von Unebenheits-Fehlstellen in einer an sich glatten Oberfläche.

Beim im Vertikalschnitt dargestellten Gegenstand 11 handelt es sich beispielsweise um einen gehobelten Balken oder um ein Gußstück aus Metall oder Kunststoff. Die Oberfläche 12 dieses Gegenstandes 11 soll an sich glatt sein und sei mit einem Muster relativ hohen Helligkeitskontrastes ausgestattet, etwa in der Form der Maserung von Holz oder in der Form eine Aufdruckes oder einer aufkaschierten Muster-Folie (in der Zeichnung nicht dargestellt). Die Qualität der Oberfläche 12 wird mittels eines Bildaufnehmers 13 kontrolliert, der quer zur Oberfläche 12 ausgerichtet ist und relativ zu welchem der Gegenstand 11 einen Vorschub 14 erfährt. Der gerade inspizierte, also vom Bildaufnehmer 13 erfaßte Bereich 15 erfährt unter spitzem Winkel zur Oberfläche 12 eine Bestrahlung 16, bei der es sich im Falle einer optischen Kamera 17 um sichtbares Licht aus wenigstens einem über der Oberfläche 12 gehalterten Scheinwerfer 18 handelt. Diese geneigte Bestrahlung 16 ruft in fehlerhaften Bereichen 15 (der glatt angestrebten Oberfläche 12) Schlagschatten 19 hinter den Streiflicht-Rändern 20 von Vertiefungen 21 und Erhöhungen 22 in der Oberfläche 12 hervor, welche etwa durch Kraterbildung aufgrund lokaler mechanischer Beschädigung entstanden, in der Zeichnung aber zur Vereinfachung der Darstellung unrealistisch abstrahiert sind.

Wenn als Bildaufnehmer 13 eine Graulicht-Kamera 17 eingesetzt ist, dann sind bei der Oberflächen-Inspektion die Schatten 19 nicht von ähnlich kontrastreichen Mustern auf der Oberfläche 12 zu unterscheiden. Diese Unterscheidung ist selbst mit einer Farb-Kamera 17 schwierig, und sie wird wieder unmöglich, wenn die Schatten-Farbe der Farbe eines Musters in der Umgebung der Fehlstelle entspricht. Deshalb wird gemäß vorliegender Erfindung gewissermaßen eine Farbcodierung von Fehlstellen-Schatten 19 vorgenommen.

Hierfür wird der zu inspizierende Bereich 15 der Oberfläche 12 aus zwei entgegengesetzten Richtungen (vorzugsweise unter gleichen Neigungswinkeln) unterschiedlich bestrahlt. Für die vorliegende Erläuterung sei der Unterschied eine rote 16.r und eine grüne 16.g Bestrahlung aus entsprechenden Scheinwerfern 18 in bzw. gegen Vorschubrichtung 14 angenommen, aber die Bestrahlungsrichtung kann auch quer zum Vorschub 14 orientiert sein. Stets hat diese Mischbestrahlung zu Folge, daß in der ansonsten ebenen Oberfläche 12 fehlstellenbedingte Schattenwürfe 19.g 19.r, die jeweils einer der Bestrahlungen 16.g 16.r zugeordnet sind, doch nicht dunkel bleiben, weil sie von der entgegengesetzten Richtung aus mit der anderen Bestrahlung 16.r bzw. 16.g aufgehellt werden. Im Inspektions-Bereich 15 erfaßt der Bildaufnehmer 13 also nur bei ungestörter Oberfläche 12 (gleichgültig ob gemustert oder nicht) gleichförmiges Mischlicht M aus den beiden unterschiedlichen Bestrahlungen 16.r und 16.g; dagegen nur reflektierte Strahlung 16.r im Schlagschatten 19.g hinter dem für die andere Strahlung 16.g wirksamen Streiflicht-Rand 20.g (und umgekehrt: Reflexion allein von der Bestrahlung 16.g aus dem Schlagschatten 19.r).

Die Reflexionen an der ebenen Oberfläche 12, gleichgültig ob sie mit Mustern ausgestattet ist oder nicht, liefern also - im Gegensatz zur Umgebung der Streiflicht-Ränder 20 von Oberflächen-Fehlstellen - gleichförmige Mischlicht-Reflexionen, im dargestellten Beispielsfalle aus roter und grüner Bestrahlung 16.r/16.g. In einer Farb-Kamera 17 können diese beiden Farbkomponenten durch einen Vergleich, durch z. B. Subtraktion voneinander oder durch Farbintensitäts-Verhältnisbildung, neutralisiert werden. Dagegen verbleiben für die Signalverarbeitung hinter der Farb-Kamera 17 die im wesentlichen ungemischten Farbreflexionen aus der roten oder aus der grünen Bestrahlung 16.r, 16.g vom Schlagschatten 20.g bzw. 20.r der jeweils anderen Bestrahlung 16.g bzw. 16.r. So wird mit der Kamera 17 ein eindeutiges Kriterium dafür gewonnen, daß es sich hier auf der Oberfläche 12 nicht um Helligkeitskontraste aufgrund von Mustern, sondern tatsächlich um mechanische Fehlstellen (Erhöhungen oder Vertiefungen) handelt. Diese Oberflächen-Fehlstellen können dann einer weiteren Klassifizierung unterzogen werden, indem z. B. das aktuelle Farbdifferenz-Kamerabild, etwa wie in der EP 0 623 885 A2 näher beschrieben, weiterverarbeitet wird.

## Patentansprüche

1. Einrichtung zum Erfassen von Fehlstellen auf einer glatten Oberflächen (12) eines Gegenstandes (11) mittels eines Bildaufnehmers (13) für Schlagschatten (19), die bei geneigter Bestrahlung (16) der Oberfläche (12) auftreten,
**dadurch gekennzeichnet**,
daß die Oberfläche (12) gleichzeitig in unterschiedlichen Richtungen wenigstens zwei von einander unterscheidbare Bestrahlungen (16) erfährt, deren neben Fehlstellen-Rändern (20) in der Oberfläche (12) auftretenden Uni-Schlagschatten (19) im Vergleich zu benachbarten Mischlichtreflexionen vom Bildaufnehmer (13) verarbeitet werden.

2. Einrichtung nach Anspruch 1,
**dadurch gekennzeichnet**,
daß zwei Bestrahlungen (16) einander entgegengerichtet, vorzugsweise unter jeweils gleichem spitzem Winkel gegenüber der Oberfläche (12), erfolgen.

3. Einrichtung nach Anspruch 2,
**dadurch gekennzeichnet**,
daß die beiden Bestrahlungen (16) in und entgegen oder quer zu der Richtung eines Vorschubs (14) des Gegenstandes (11) relativ zum Bildaufnehmer (13) erfolgen.

4. Einrichtung nach einem der vorangehenden Ansprüche,
**dadurch gekennzeichnet**,
daß der Bildaufnehmer (13) mit einer Farb-Kamera (17) ausgestattet ist und die Oberfläche (12) Bestrahlungen (16) in unterschiedlichen Farben, vorzugsweise in rot und grün, erfährt, welche von glatter Oberfläche (12) als Mischlicht (M) zum Bildaufnehmer (13) reflektiert werden.

5. Einrichtung nach Anspruch 4,
**dadurch gekennzeichnet**,
daß in oder nach der Farb-Kamera (17) ein Vergleich, z. B. Subtraktion oder Verhältnisbildung, der Signalanteile der beiden unterschiedlich eingefärbten Bestrahlungen (16) im Reflex-Mischlicht (M) von der ebenen Oberfläche (12) erfolgt.

6. Einrichtung nach einem der Ansprüche 1 bis 3,
**dadurch gekennzeichnet**,
daß zwei Bestrahlungen (16) mit Licht unterschiedlicher Polarisierung oder Modemeigenschaft erfolgen und die Reflexionen von der Oberfläche (12) über einen Analysator vom Aufnehmers (13) erfaßt werden.
